# EUROPEAN PATENT SPECIFICATION

(11) **EP 2 714 676 B1**
(45) Date of publication and mention of the grant of the patent: **24.04.2019**
(21) Application number: 12723214.8
(22) Date of filing: 25.05.2012
(51) Int. Cl.: C07D 401/04

(54) **A PROCESS FOR THE PREPARATION OF POLYMORPHIC FORM I OF ETORICOXIB**
VERFAHREN ZUR HERSTELLUNG EINER POLYMORPHEN FORM I VON ETORICOXIB
PROCÉDÉ POUR LA PRÉPARATION DE LA FORME POLYMORPHE I DE L'ÉTORICOXIB

(30) Priority: 27.05.2011 SI 201100190
(43) Date of publication of application: 09.04.2014
(73) Proprietor: Farma GRS, d.o.o., 8000 Novo mesto (SI)
(72) Inventor: KLJAJIC, Alen, 3000 Celje (SI); ZUPANCIC, Silvo, 8000 Novo mesto (SI); VRBINC, Miha, 8000 Novo mesto (SI); TROST, Sabina, 1330 Kocevje (SI)
(74) Representative: Hoffmann Eitle
(86) International application number: PCT/EP2012/059852
(87) International publication number: WO 2012/163839

(56) References cited:
- WO-A1-01/37833
- WO-A1-02/096877
- WO-A2-2010/097802
- CHAD R. DALTON ET AL: "Investigating the hydrate conversion propensity of different etoricoxib lots", JOURNAL OF PHARMACEUTICAL SCIENCES, vol. 95, no. 1, 1 January 2005 (2005-01-01), pages 56-69, XP055033301, ISSN: 0022-3549, DOI: 10.1002/jps.20499

## Description

### Field of the invention

Present invention relates to a process for preparation of polymorphic form I of etoricoxib. The polymorphic form of etoricoxib obtained by the process of the present invention is characterized by high polymorphic purity and high chemical and physical stability.

### Background of the invention

Etoricoxib is a common name for 5-chloro-6'-methyl-3-[4-(methylsulfonyl)phenyl]-2,3'-bipyridine, a compound of formula (I):

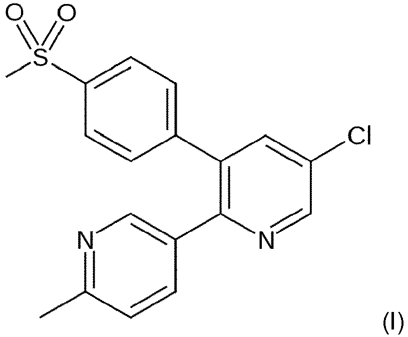

Etoricoxib belongs to the class of COX-2 selective inhibitors and is particularly used for the treatment rheumatoid arthritis, psoriatic arthritis, osteoarthritis, ankylosing spondylitis, chronic low back pain, acute pain and gout. Like other COX-2 inhibitors, its mechanism of action is by reduction the generation of prostaglandins (PGs) from arachidonic acid.

Etoricoxib is available in the market under trademark Arcoxia, as a film coated tablet in 30mg, 60mg, 90mg and 120mg strengths.

Several Etoricoxib polymorphic forms (Forms I-V; Forms IX-XVI), two hydrate forms and amorphous form have been reported.

Polymorphic forms I-IV, hemihydrate form, sesquihydrate form and amorphous form have been disclosed in WO 2001/037833.

WO 02/096877 and WO 01/092230 disclose polymorphic form V and mixtures thereof with other polymorphic forms.

WO 2005/085199 describes polymorphic forms IX to XVI.

It is reported in J. Phar. Sci, 95, 11, 2006 that spontaneous and uncontrolled crystallization can produce any of several etoricoxib polymorphs or mixtures of them. It is suggested that for obtaining the desired polymorph the crystallization process had to be optimized and controlled by seeding. However, process irregularity such as etoricoxib concentration outside of the desired range could result in formation of undesired polymorph.

In J. Pharm. Sci., 95, 56-69, 2006 it is reported that polymorphic forms of etoricoxib show conversion to hydrate form which can depend on particle size, amount/type of impurity, undetectable amorphous content which can catalyze conversion to hydrate, presence of initial hemihydrate seeds.

It is evident from the prior art processes that solely by selection of a solvent the desired polymorphic form could not be obtained. In WO 2005/085199 it is reported, see Example 6, that crystallization from 2-propanol provides etoricoxib in form XIII, which is a mixture of form I with minimal amounts of form IV, whereas according to a process disclosed in WO2010/097802, see Example 26, crystallization from 2-propanol results, according to XRDP provided, in formation of etoricoxib in a mixture of polymorphic form IV with trace amounts of form I.

Consequently, there is still a need for an improved, reliable, well defined process which would be reproducible in large scale to prepare polymorphic form I in high yields, high purity and in absence of any traces of non-desired forms.

Polymorphic forms are of particular interest due to the provision of essentially pure compounds permitting an exact characterization of physico-chemical properties and possible favorable pharmacological characteristics, such as improved solubility, stability and particle size. There is, however, still a need for a robust process for preparation of polymorphic form essentially free of other polymorphic forms, hydrate forms and amorphous form.

It has been reported that forms IV and V are more thermodynamically stable than forms I-III, but we have surprisingly found out that form I prepared in essentially pure form resists to any conversion and is stable under technological process conditions such as milling, compression or blending with excipients. Another advantage of form I is better solubility in comparison to polymorphic forms IV and V which can afford to improved pharmacological properties such as bioavailability.

Accordingly an object of the present invention resides in the provision of a robust, process for preparation a polymorphic form of form I in essentially pure form in high yields, exhibiting desired pharmacological properties for the preparation of stable pharmaceutical compositions.

### Figures

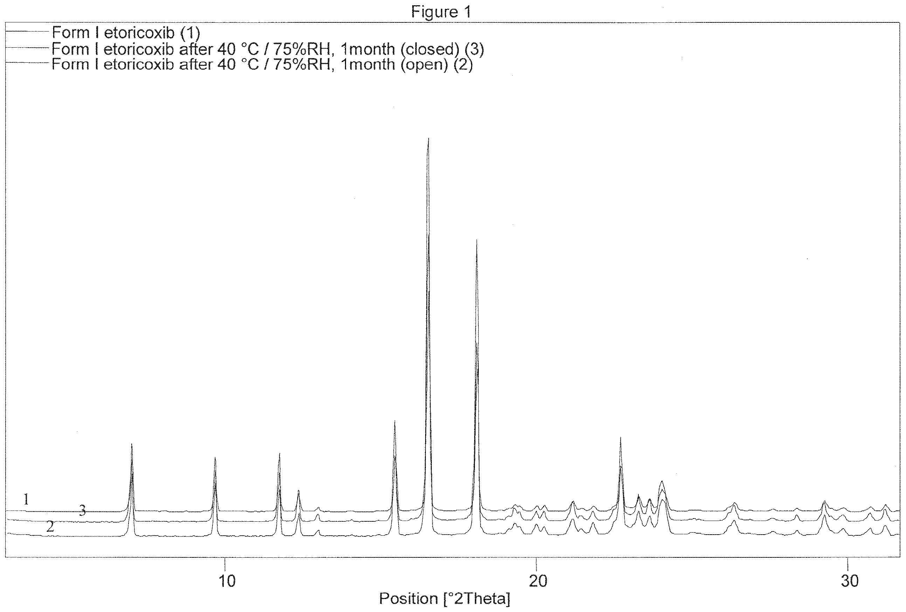

Figure 1 shows an X-ray diffraction pattern (XRDP) of etoricoxib polymorphic form I (1); XRDP of polymorphic form I after storage at 40°C/75%RH/1 month, open (2) and XRDP of polymorphic form I after storage 40°C/75%RH/1month, closed (3)

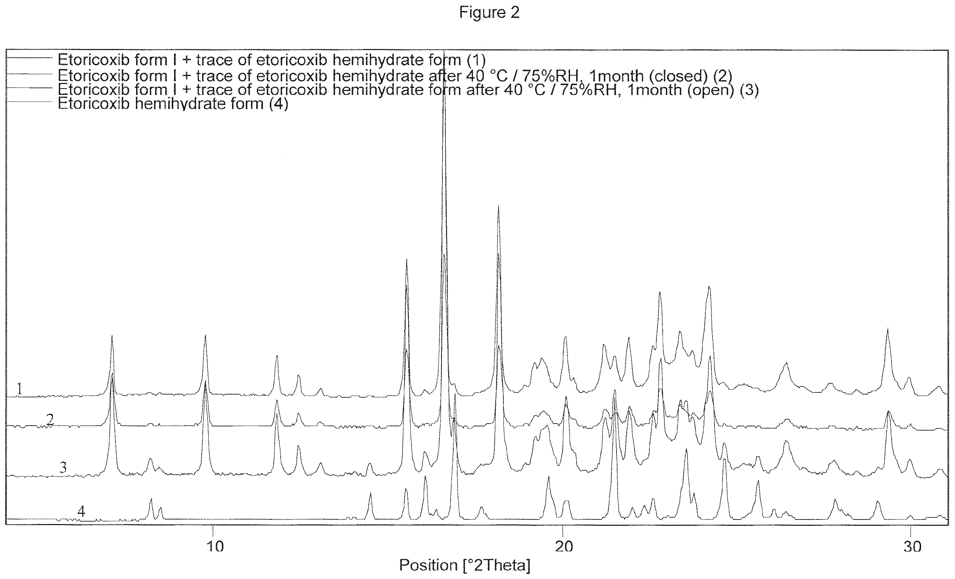

Figure 2 shows XRDP of etoricoxib polymorphic form I with trace of hemihydrate form (1); XRDP of polymorphic form I with trace of hemihydrate form after storage at 40°C/75%RH/1 month, closed (2) and XRDP of polymorphic form I with trace of hemihydrate form after storage 40°C/75%RH/ 1 month, open (3)

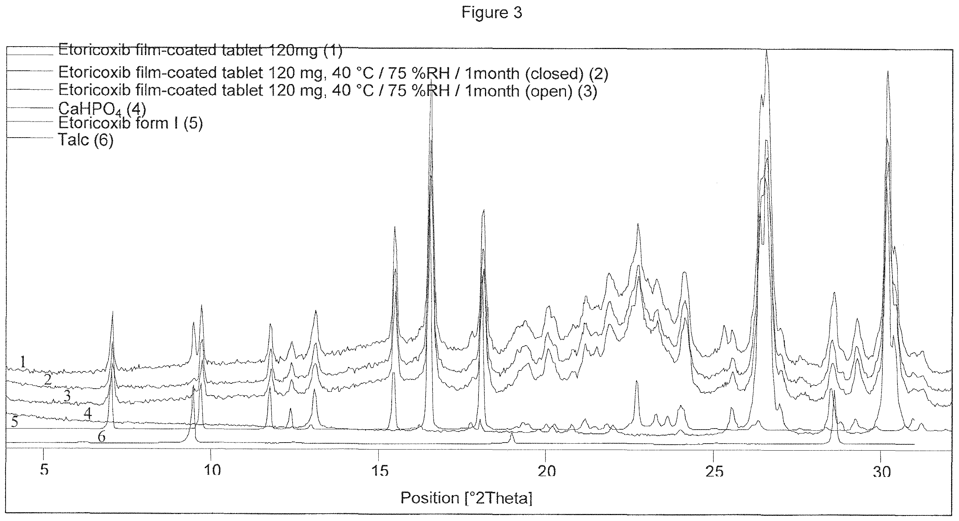

Figure 3 shows XRDP of etoricoxib film coated tablet comprising etoricoxib polymorphic form I (1); XRDP of etoricoxib film coated tablet comprising etoricoxib polymorphic form I after storage at 40°C/75%RH/ 1 month, closed (2); XRDP of etoricoxib film coated tablet comprising etoricoxib polymorphic form I after storage at 40°C/75%RH/ 1month, open (3); XRDP of CaHPO₃ (4); XRDP of etoricoxib polymorphic form I (5); XRDP of talc (6)

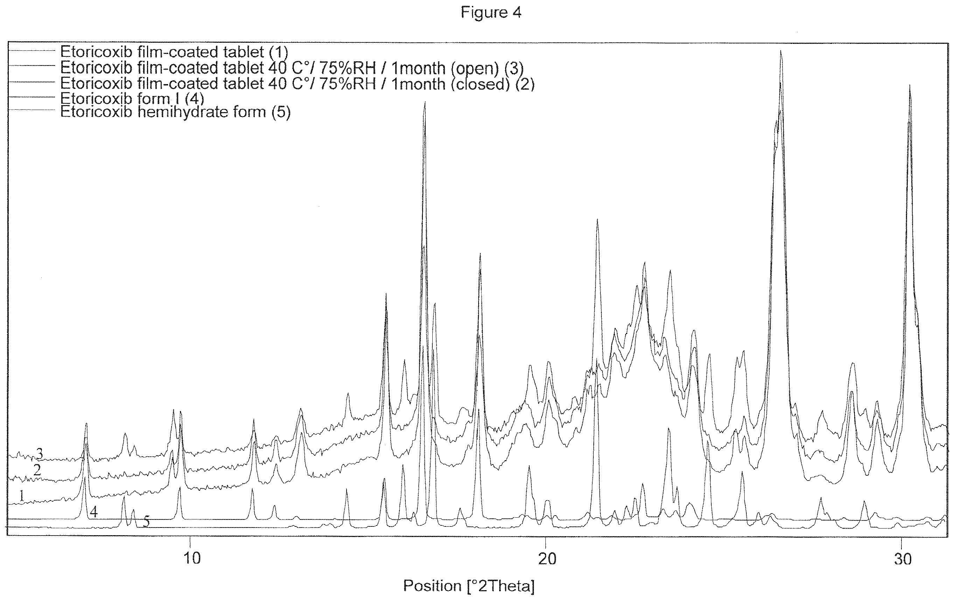

Figure 4 shows XRDP of etoricoxib film coated tablet comprising etoricoxib polymorphic form I with trace of hemihydrate form (1); XRDP of etoricoxib film coated tablet comprising etoricoxib polymorphic form I with trace of hemihydrate form after storage at 40°C/75%RH/1 month, closed (2); XRDP of etoricoxib film coated tablet comprising etoricoxib polymorphic form I with trace of hemihydrate form after storage at 40°C/75%RH/1 month, open (3); XRDP of etoricoxib polymorphic form I (4); XRDP of etoricoxib hemihydrate form (5)

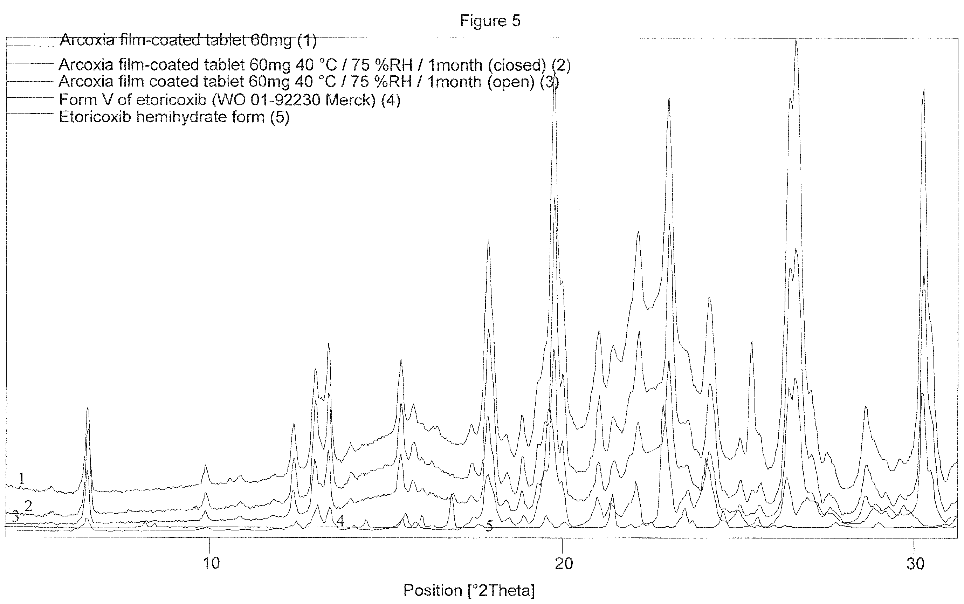

Figure 5 shows XRPD of Arcoxia film coated tablet 60mg (1);); XRDP of Arcoxia film coated tablet 60mg after storage at 40°C/75%RH/1 month, closed (2); XRDP of Arcoxia film coated tablet after storage at 40°C/75%RH/1 month, open (3); XRDP of etoricoxib polymorphic form V (4); XRDP of etoricoxib hemihydrate form (5)

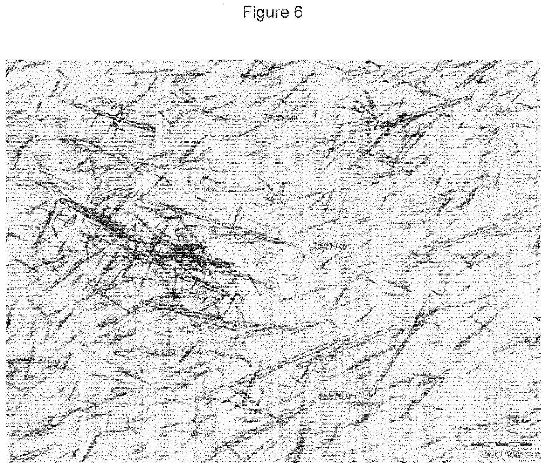

Figure 6 shows a microscope picture of etoricoxib polymorphic form I obtained by a process according to present invention

### Detailed description of the invention

According to the present invention a process for the preparation of a stable and crystallographically essentially pure polymorphic form I of etoricoxib is provided.

The term "crystallographically essentially pure" means that polymorphic form comprises less than 2 wt %, preferably less than 1%; more preferably the form is free of any other polymorphic form, amorphous form or solvate form. Polymorphic purity has been determined by means of X-ray powder diffraction.

The process for the preparation of crystallographically essentially pure polymorphic form I of etoricoxib according to present invention comprises:
(i) dissolving etoricoxib in a suitable solvent system at a temperature in the range of from 50°C to refluxing temperature of said organic solvent system;
(ii) optionally filtering the obtained crystallization mixture;
(iii) cooling the crystallization mixture to a nucleation temperature in the range of from 30°C to 56°C,
(iv) then cooling the crystallization mixture to a temperature in the range of from -20°C to 28°C,
(v) separating and drying the crystallized polymorphic form I of etoricoxib;
wherein polymorphic purity is determined by means of X-ray powder diffraction, wherein polymorphic form I of etoricoxib is characterized by an X-ray diffraction pattern obtained using a Cu K alpha source and comprising signals with peak positions in accordance with Figure 1; and
wherein the solvent system is 2-propanol.

The term "nucleation temperature" as used herein relates to the temperature of the crystallization mixture at which the nucleation is induced i.e. the temperature at the onset of the crystallization.

Etoricoxib is preferably dissolved in the solvent system (2-propanol) at a temperature in the range between 50°C and the refluxing temperature of the solvent system, preferably between 60°C and the refluxing temperature and more preferably between 70°C and the refluxing temperature, suitably the etoricoxib is dissolved at the refluxing temperature.

The term "refluxing temperature of a solvent system" as used herein relates to a temperature in the range of the boiling temperature of a particular solvent to the boiling temperature plus 15°C, i.e. the reflux temperature of methanol is in the range of from 78°C to 93°C.

The amounts of etoricoxib and solvent used are preferably corresponding to a solute:solvent weight ratio of less than 1:20, more preferred less than 1:10, even more preferred less than 1: 7 and most preferred less than 1:6.

In the step (iii) the crystallization mixture, i.e. a solution of etoricoxib in the solvent system, is gradually cooled down to a nucleation temperature of the crystallization mixture maintaining a controlled cooling profile at average cooling rate within 0.1 to 2.5°C/min. Nucleation temperature can be selected within the range of 30° to 56°C, preferably within the range of 45° to 55°C.

There are two common types of nucleation mechanisms. Primary (homogeneous) nucleation occurs at the onset of crystallization, when the concentration of the solvent exceeds the metastable region, and secondary nucleation, which is caused by contacts between a crystal and another surface, and occurs within the metastable region. Crystal-to-crystal and crystal-to-impeller contacts are the most common sources of secondary nucleation. Secondary nucleation is therefore affected by the mixing energy input to the crystallizer.

Nucleation process in the context of the present invention can be induced either by primary and/or secondary nucleation mechanism.

Primary nucleation in step (iii) can be affected by stirring the crystallization mixture at specified nucleation temperatures as long as the precipitation of crystals is initiated.

In case of secondary nucleation the crystallization batch is seeded by addition of crystals of etoricoxib in pure polymorphic form I. The crystallization batch is seeded by addition of crystals of etoricoxib at least once during the cooling time when the temperature of crystallization batch is within above defined nucleation temperature range in order to avoid excessive supersaturation with respect to etoricoxib and resulting spontaneous crystallization into non-desired polymorphic forms. The seeding may be repeated in order to ensure constant presence of crystalline etoricoxib during the cooling, suitably the crystallization batch can be seeded semi-continuously until crystallization has started. The seeds of etoricoxib can be added in powder form or in form of suspension in any type of liquid.

The obtained suspension of etoricoxib crystallization mixture after step (iii) is gradually cooled down to the temperature, at which the crystals will be isolated from the mother liquor i.e. final crystallization temperature, in the range of (-20) to 28°C, preferably 0 to 15°C, and more preferred 7 to 15°C maintaining a controlled cooling profile.

"Final crystallization temperature" in the context of this invention means the lowest end temperature during the crystallization process. This is usually the temperature of formed suspension immediately before the isolation process is performed.

Preferably the average cooling rate does not exceed 2.0° C/min, more preferably the average cooling rate is kept within the range of 0.5° to 2°C/min, most preferably within the range of 1°-1.5°C/min. Suitably the average cooling rate may be kept in this range for the entire cooling; comprising initial cooling prior to nucleation temperature is reached (step iii) and cooling to final crystallization temperature (step iv).

In the examples the temperature of the crystallization mixture versus time during the cooling process is presented as a linear function, but is not limited thereto. It is used as an average cooling rate for describing the approximate rate for cooling the mixture in the reactor to the desired temperature. The real function of the temperature of the crystallization mixture versus time of crystallization process can be represented by any other continuous function.

The crystallization batch is kept at final crystallization temperature for a holding time for crystal formation for at least 10 minutes, preferably in the range of 10 minutes to 10 hours and more preferred 0.5 to 2 hours. After said holding time at the final crystallization temperature, the crystalline particles of etoricoxib are isolated from the mother liquor using conventional separation techniques, e. g. filtration or centrifugation.

Preferably final crystallization temperature is 2 to 50 °C below the temperature of nucleation.

It is particularly preferred to carry out the process of the present invention by combining the preferred features described above. For instance, it is preferred to carry out the process of the invention using 2-propanol in a manner such that the nucleation temperature is within the range of 45-55°C. It is similarly preferred to carry out the process of the invention using 2-propanol and employing a cooling rate for both cooling steps within the range of 0.5 to 2°C/min and even more preferably 1 to 1.5°C/min. It is similarly preferred to carry out the process of the invention using 2-propanol and employing seeding with form I crystals for nucleation. Relying on the above-mentioned preferred nucleation temperature of 45-55°C in combination with the use of a cooling rate of 0.5 to 2°C/min and even more preferably 1 to 1.5°C (for both cooling steps) is also particularly preferred. The preferred cooling rate of 0.5 to 2°C/min and even more preferably 1 to 1.5°C (for both cooling steps) may also advantageously be combined with the measure of seeding. Similarly, seeding can also preferably be combined with the above-mentioned preferred nucleation temperature of 45-55°C.

Of course, it is even more preferred to combine three or more of these preferred features, i.e.
- use of 2-propanol, nucleation temperature of 45-55°C, and cooling temperature 0.5-2°C/min,
- use of 2-propanol, nucleation temperature of 45-55°C, and cooling temperature 1-1.5°C/min,
- use of 2-propanol, cooling temperature 0.5-2°C/min and use of seeding,
- use of 2-propanol, cooling temperature 1-1.5°C/min and use of seeding,
- use of 2-propanol, nucleation temperature of 45-55°C, and seeding,
- use of 2-propanol, nucleation temperature of 45-55°C, and seeding,
- nucleation temperature of 45-55°C, cooling temperature 0.5-2°C/min and use of seeding,
- nucleation temperature of 45-55°C, cooling temperature 1-1.5°C/min and use of seeding, and especially
- use of 2-propanol, nucleation temperature of 45-55°C, cooling temperature 0.5-2°C/min, and seeding,
- use of 2-propanol, nucleation temperature of 45-55°C, cooling temperature 1-1.5°C/min, and seeding.

Stirring of the crystallization batch is hereby performed either by magnetic or mechanical stirrer. In large scale batches preferably mechanical stirrer is used. The mechanical stirrer can be selected from impeller, propeller, turbine, paddle or anchor type configuration. During small scale crystallization processes of the present invention with or without seeding, it is preferred to use a constant stirring speed of 100 to 600 rpm. The use of lower stirring speeds of 10 to 200 rpm is preferred for larger scale processes.

Drying as used herein refers to the removal of solvent residues performed for example by heating and/or vacuum.

Another advantage of the process according the present invention is in high yield which is typically more than 80%, preferably more than 90%.

Further, the purity of the material obtained by the process of the present invention is more than 98 area% determined by HPLC, preferably more than 99 area% determined by HPLC. It is however preferred to use a starting material having a purity of more than 90 area% by HPLC and even more preferred to use a starting material with more than 95 area% by HPLC when carrying out the process of the present invention.

Moreover, the process according to this invention can be used in large scale production. It has been confirmed that the process performed on the large scale reliably and in high yield provides etoricoxib in pure polymorphic form I.

The process according to present invention yields homogenous particles in the form of readily filterable individual needles, which are suitable to be incorporated directly into the formulation. However the obtained product may be further subjected to grinding, milling or micronisation if required.

For the milling purposes fluid energy mill, jet mill, ball mill, roller mill or hammer mill are commonly used as milling equipment.

A fluid energy mill, or "micronizer" , is an especially preferred type of mill for its ability to produce particles of small size in a narrow size distribution, i. e., micronized material. As those skilled in the art are aware, micronizers use the kinetic energy of collision between particles suspended in a rapidly moving fluid (typically air or nitrogen) stream to cleave the particles. A jet mill is a preferred fluid energy mill. The suspended particles are injected under pressure into a recirculating particle stream. Smaller particles are carried aloft inside the mill and swept into a vent connected to a particle size classifier such as a cyclone. The feedstock should first be milled to about 30 µm to 100 µm which may be done using a conventional ball, roller, or hammer mill.

The starting material, in particular the material comprising or consisting of etoricoxib and to be adjusted in size, for example by milling, may have an average particle size of about 50 µm to 600 µm.

The material is fed into the micronization system in a controlled feed rate by means of a screw feeder or a vibratory feeder. The jet mill is operated with controlled air or nitrogen pressures. For the micronizer Jetmill MC-500 , the feed rate is 3 - 40 kg/hr, the Feed air /nitrogen pressure is 0.5 - 6 bar and the grinding air is 1 -6 bar.

Micronization can also be accomplished with a impact pin mill. The starting material may have an average particle size of about 20-100 microns. The material is fed into the mill system in a controlled feed rate by means of a screw feeder or a vibratory feeder. The mill is operated with controlled speed. For the Alpine UPZ 100, the feed rate is 6 - 40 kg/hr, the mill speed is 7000-15000 rpm.

The polymorphic form I of the present invention is further characterized by a small particle size determined by average particle size between 1 and 200 µm, preferably 3-100 µm, most preferably 5-40 µm, which may be determined by a laser method using a Malvern Mastersizer.

The term "average particle size" as used herein refers to the volume mean diameter of particles. The diameter and volume mean diameter can be determined by laser light scattering using e.g. a Malvern-Mastersizer Apparatus MS 2000. Particle sizes are determined by measuring the angular distribution of laser light scattered by a homogeneous suspension of particles. The particles to be subjected to the particle size measurement are first suspended in appropriate non -polar dispersant and then subjected to a size determination in a Malvern Mastersizer MS 2000 instrument. Usually, 100-800 mg of substance is dispersed in 5-10 ml of dispersant. Average particle size of etoricoxib is determined by Malvern Mastersizer 2000 Hydro F using Silicone Fluid F10 as the dispersion medium.

The process according to present invention is a robust, reproducible and scalable crystallization process that generates etoricoxib in pure crystalline form I. Constant polymorphic purity in both the drug substance and in drug product as well as controlling the amorphous content in the drug substance assures product quality and reliability.

Etoricoxib in polymorphic form I as obtained by a process according to present invention is surprisingly stable by means of chemical and physical stability. We have surprisingly found out that the polymorphic form I according to present invention does not convert to other polymorphic form when subjected to stress stability conditions at 40°C/75%RH for 1 month in open or closed container, whereas in case polymorphic form I comprising traces of hemihydrate is subjected to stress stability conditions the content of hemihydrate increases.

Furthermore, the polymorphic form according to the present invention retains its chemical and physical properties also when incorporated into the pharmaceutical composition. The content of total impurities does not increase when the pharmaceutical composition containing pure polymorphic form I is subjected to stress conditions. Moreover the physical form of polymorphic form I according to present invention does not change when subjected to stress conditions whereas this is not the case for polymorphic form I containing trace of hemyhidrate and for polymorphic form V incorporated into commercially available Arcoxia tablets, which under stress stability testing convert at least partially into other polymorphic forms. Results on stress stability testing are explained more in details in Experimental section.

Etoricoxib used in the respective processes for the preparation of the polymorphic form I may be prepared according to any method known to the skilled person. The preparation of etoricoxib is outlined for example in US 5861419, EP912518, EP1071745 or WO2010/097802.

The etoricoxib prepared by the process according to the present invention can be used as active ingredient in pharmaceutical compositions or formulations. Of particular advantage is that the present polymorph form is surprisingly stable when subjected to a standard technological processes used in the preparation of pharmaceutical formulations.

The pharmaceutical compositions are preferably solid pharmaceutical compositions comprising polymorphic form I of etoricoxib together with excipients.

In a further aspect the composition is such that the average particle size of etoricoxib is between 1 and 200 µm, preferably 3-100 µm, most preferably 5-40 µm. The average particle size may be determined by a laser method using a Malvern Mastersizer.

The solid pharmaceutical composition comprises etoricoxib and excipients. The compositions can be in the form of tablets, minitablets, microtablets, coated tablets, coated minitablets, coated microtablets, pills, powders, lozenges, sachets, soft and hard gelatine capsules, suppositories etc. The dosage form is preferably suitable for oral application.

The composition is preferably formulated in a unit dosage form, each dosage containing about 10 to about 200 mg of active ingredient, furthermore described as "etoricoxib", more usually about 30 mg to about 120 mg of etoricoxib. The term "etoricoxib" includes etoricoxib in pure polymorphic form I. The preferred composition comprise active ingredient etoricoxib of polymorphic form I, which retains its form during stress stability testing which indicated the stability of the incorporated physical form of etoricoxib during product's shelf-life.

The term "unit dosage form" refers to physically discrete units suitable as unitary dosages for human subjects and other mammals, each unit containing a predetermined quantity of etoricoxib calculated to produce the desired therapeutic effect, in association with one or more suitable excipients. An immediate release composition of unit dosage form is preferred.

The excipients used for the preparation of the pharmaceutical formulations particularly include excipients with different functions, for example one or more diluents, one or more binders, one or more disintegrants, one or more lubricants, one or more glidants and one or more antitacking agents. Other and further excipients with different functions, e.g. pH modifiers, antioxidants, surfactants, etc.) can also be included in the composition and the opposite, one or more excipients with different functions can be excluded from the composition. Furthermore, excipients can have multiple functions, i.e. one excipient is used as diluent and/or binder and/or disintegrant etc.

The composition comprise one or more diluents, selected form carbohydrates such as lactose, e.g. lactose monohydrate, anhydrous lactose, spray dried and/or granulated lactose, sucrose, fructose, dextrates, saccharose, raffinose, trehalose, fructose or mixtures thereof, dextrin, sugar alcohols such as xylitol, mannitol, maltitol, isomalt, sorbitol, powdered cellulose, microcrystalline cellulose, silicified microcrystalline cellulose, low moisture starch, corn starch, pregelatinised starch, low moisture pregelatinised starch, calcium salts of phosphoric acid such as calcium hydrogen phosphate in anhydrous and hydrated form, calcium, sodium or potassium carbonate or hydrogen carbonate and calcium lactate or mixture of diluents. Preferably, low moisture diluents are applied. In another aspect, diluents are preferably selected from microcrystalline cellulose, calcium hydrogen phosphate, sugars and polyols or mixtures thereof. More preferably, the composition comprises at least one diluent, selected from microcrystalline cellulose and calcium hydrogen phosphate or mixtures thereof.

The composition furthermore comprise one or more binders, selected from povidone (polyvinylpyrrolidone), copovidone (vinylpyrrolidone-vinyl copolymer), powdered cellulose, crystalline cellulose, microcrystalline cellulose, siliconised microcrystalline cellulose, cellulose derivatives such as hydroxymethylcellulose, hydroxyethylcellulose, hydroxypropylcellulose (hyprollose), low substituted hydroxypropylcellulose and hydroxypropylmethylcellulose (hydpromellose) or other cellulose ethers, corn or potatato or rice starch, pregelatinised starch, α-starch pr dextrin, gum arabic, pullulan, polymethacrylates or mixture of binders. Preferably, the dry binders can be selected form copovidone, povidone, hyprmellose, microcrystalline cellulose, starch derivatives such as pregelatinised starch or low substituted hydroxypropylcellulose or mixture thereof. More preferably, the composition comprises at least one binder, selected from microcrystalline cellulose, povidone and hydroxypropylcellulose or mixtures thereof.

The composition furthermore comprise one or more disintegrants, selected from crospovidone, starch, pregelatinised starch, sodium starch glacolate, hydroxypropyl starch, microcrystalline cellulose, carboxymethylcellulose sodium and/or calcium, cross-linked carboxymethylcellulose sodium, carboxymethylcellulose calcium, polacrilin potassium, low substituted hydroxypropylcellulose, sodium and/or calcium alginate, docusate sodium, methylcellulose, agar, guar gum, chitosan, alginic acid or mixtures thereof. Preferably, disintegrants are selected from microcrystalline cellulose, croscarmellose sodium, low substituted hydroxypropylcellulose and crospovidone or mixtures thereof.

The composition furthermore comprise one or more lubricants, selected from the group of metal salts of fatty acids with 12 to 20 carbon atoms such as magnesium, calcium, aluminium or zinc stearate, magnesium palmitate and magnesium oleate, hydrogenated vegetable oil, hydrogenated castor oil, talc, meads wax or spermaceti, boric acid, sodium stearyl fumarate, macrogols or mixtures thereof. Preferably, lubricants are selected from magnesium and/or calcium stearate, talc or mixtures thereof.

The composition can furthermore comprise one or more glidants, selected from colloidal silica (Aerosil 200 or Aerosil 072) and magnesium trisilicate.

The composition can furthermore comprise one or more antitacking agents, selected from talc, glyceryolmonostearate or stearic acid.

The compositions of the tablet core preferably comprise etoricoxib and at least one excipient selected from excipients with the function as defined: diluent, binder, disintegrant and lubricant. In another aspect, the compositions of the tablet core preferably comprise etoricoxib and at least one excipient selected from excipients with the function as defined: diluent, binder, disintegrant, lubricant and/or glidant. In yet another aspect, the compositions of the tablet core preferably comprise etoricoxib and at least one excipient selected from excipients with the function as defined: diluent, binder, disintegrant, lubricant and antitacking agent. In yet another aspect, the compositions of the tablet core preferably comprise etoricoxib and at least one excipient selected from excipients with the function as defined: diluent, binder, disintegrant, lubricant, glidant and antitacking agent.

If not indicated otherwise, all percentages given herein are wt. %, based on the total weight of the tablet core without film coating layer. Preferably, the formulations comprise etoricoxib in an amount of 15-60%, more preferably 20-50%, even more preferably 20-40%.

According to a more preferred aspect, the composition comprise:

| | |
|---|---|
| etoricoxib: | 15-60%, more preferably 20-50%, even more preferably 20-40%; |
| diluent: | 20-80%, more preferably 30-80%, even more preferably 35-70%; |
| binder: | 1-80%, more preferably 1-60%, even more preferably 1-50%; |
| disintegrant: | 1-80%, more preferably 2-60%, even more preferably 2-50%; |
| lubricant: | 1-20%, more preferably 1-15%, even more preferably 1-10%; |
| glidant: | 0-20%, more preferably 0-15%, even more preferably 0-10%; |
| antitacking agent: | 0-20%, more preferably 0-15%, even more preferably 0-10%; |

Optionally, cores/tablets can be coated with conventional materials used for film coating, i.e. as described in Pharmaceutical Coating Technology, 1995, edited by Graham Cole. Film coating formulations usually contain the following components: polymer(s), plasticizer(s), colourant(s)/opacifier(s), vehicle(s). In film coating suspension the minor quantities of flavours, surfactants and waxes can be used. The majority of the polymers used in film coating are either cellulose derivatives, such as the cellulose ethers, or acrylic polymers and copolymers. Occasionally encountered are high molecular weight polyethylene glycols, polyvinyl pyrrolidone, polyvinyl alcohol and waxy materials.

Typical cellulose ethers are hydroxyethylcellulose, hydroxypropylcellulose, hydroxypropylmethylcellulose, methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration/dissolution of the film.

The commonly used plasticizers can be categorized into three groups: polyols (glycerol, propylene glycol, macrogols), organic esters (phthalate esters, dibutyl sebacetate, citrate esters, triacetin), oils/glycerides (castor oil, acetylated monoglycerides, fractionated coconut oil).

Colourants/opacifiers are classified into several groups: organic dyes and their lakes, inorganic colours, natural colours. Combination of different materials form each group can be combined in defined ratios. Film coating suspensions can be used as ready-to-make preparations which are available on the market.

Film coating dispersion can be prepared by using different solvents (water, alcohols, ketones, esters, chlorinated hydrocarbons), preferably water.

A composition of coating suspension (calculated on dry material) is particularly preferred which comprises:
- 1-99% by weight of polymer, preferably 1-95% of polymer,
- 1-50% by weight of plasticizer, preferably 1-40% of plasticizer,
- 0.1-20% of colourant/opacifier, preferably 0.1-10% of colourant/opacifier.

The compositions of the coating layer preferably comprise at least one excipient selected from excipients with the function as defined of polymer and plasticizer. In another aspect, the composition of the coating layer preferably comprise at least one excipient selected from excipients with the function as defined polymer, plasticizer and colorant/opacifier.

The composition is prepared by known technological procedures, e.g. direct compression or granulation, using well known and readily available excipients. In another aspect, the composition is prepared in a manner known per se, for example by means of conventional mixing, granulating, coating, suspensing or lyophilizing processes. In the preparation of the composition, the active ingredient will usually be mixed with at least one excipient or a mixture of excipients, or diluted by an excipient or mixture of excipients, or enclosed within an excipient or a mixture of excipients. When at least one excipient serves as a diluent, it may be a solid, semisolid or liquid material which acts as a vehicle or medium for the etoricoxib.

Based on the knowledge of the properties and stability of the etoricoxib, it is moisture sensitive drug. Therefore, a process for the preparation of the composition comprises the steps, which are preferred to exclude the influence of water in each technological step. This means that the processes are selected between the direct compression, dry granulation or wet granulation.

The composition can be prepared by direct compression by processes known in the art.

The "dry granulation" method is a method of formulation wherein the raw material powder is subjecting a compression moulding into a pellet or sheet, using granules produced by crushing and division by a suitable method. Such methods are described in "Theory and Practice of Industrial Pharmacy", 3rd ed. (Lachmann L et al, Lea&Febiger, 1986) or "Pharmaceutical Dosage Forms: Tablets, Volume 1", 2nd ed (Lieberman HA et al, Marcel Dekker, 1989).

Dry granulation can be performed by processes known in the art as slugging and/or roller compaction, the latter being preferred. Etoricoxib, optionally sieved to eliminate agglomerates, is usually mixed with at least one excipient or a mixture of excipients into a compactat/comprimate by using roller compactor or compression machine. The obtained compactat/comprimate is crushed and optionally sieved to obtain a uniform distribution of the granules of a dry granulate.

"Wet granulation" can be performed by using granulation liquids, based on water, organic solvents or organic/water liquid solvents, which contain less that 50% of water. Organic solvents used in granulation for the preparation of the composition are selected form alcohols with 1 to 4 carbon atoms like absolute ethanol, concentrated alcohol (96 vol%), methanol, isopropropanol, ketons such as acetone or esters such as ethylacetate or mixtures thereof as solvents or as organic/water dispersions, containing less that 50% of water. Excipients selected from diluents, binders, disintegrants, lubricants, glidants and antitacking agents can optionally be dissolved, suspended, emulsified or dispersed into the granulation liquid which is sprayed onto the powder mixture comprising etoricoxib and at least one excipient or a mixture of excipients. Granulation could be performed using state of the art granulators such as high shear granulator, low shear granulator, or a fluid bed granulator.

In the preparation of granulate, either by wet granulation or dry granulation, etoricoxib can be divided between granulate, i.e. is incorporated intragranularly, or is added partially as an additive to the granulate, i.e. extragranularly, alone or in combination with at least one excipient or a mixture of excipients. In another aspect, at least one excipient or a mixture of excipients can be divided between granulate or as an additive to the granulate.

The mixing of etoricoxib and at least one excipient or a mixture of excipients may be effected in conventional devices used for mixing of powder, e.g. motionless (passive) mixers, fluidized bed, diffusion, biconic diffusion, biconic, tubular, cubic, planetary, Y-, V-shaped or high-shear mixers. The same mixing equipment may be used also in the preparation of the compression mixture in direct compression. In another aspect, the same equipment may be used in the preparation of compression mixture with the prior step of a granulate preparation by a granulation as described by the terms "wet granulation" and "dry granulation".

For the wet granulation, conventional drying devices such as a fluid-bed dryer or drying chambers can be used.

In the processes, the compression, in particular to tablet or tablet cores, can be effected using an automatic rotary compressing machine from different manufacturers of equipment for use in pharmaceutical industry.

Conventional equipment can be used for applying a film coating, such as a Wurster coating system or conventional coating pans for use in pharmaceutical industry.

The process for preparing the pharmaceutical compositioncan be carried out as a granulation process or direct compression process. In each case, etoricoxib is first prepared according to a suitable synthetic process and then purified, e.g. by crystallization or other means. Then, the size of etoricoxib particles is determined and if it is found that there are particles having a suitable particle size, then this etoricoxib is milled or crunched to a smaller size, i.e. that the average particle size of etoricoxib is between 1 and 200 µm, preferably 3-100 µm, most preferably 5-40 µm. The average particle size is determined by laser method on Malvern Mastersizer.

In a further aspect, a direct compression process comprises:
- mixing of etoricoxib and excipient or a mixture of excipients to give compression mixture;
- compressing the compression mixture to the desired form;
- optionally, applying a coating.

In another aspect, the dry granulation process comprises:
- granulating an excipient or a mixture of excipients using processes known in the art as slugging and/or roller compaction;
- addition of etoricoxib and excipient or a mixture of excipients to the granulate to give compression mixture;
- compressing the compression mixture to the desired form;
- optionally, applying a coating.

In another aspect, the dry granulation process comprises:
- granulating etoricoxib and excipient or a mixture of excipients using processes known in the art as slugging and/or roller compaction;
- addition of excipient or a mixture of excipients to the granulate to give compression mixture;
- compressing the compression mixture to the desired form;
- optionally, applying a coating.

In another aspect, the dry granulation process comprises:
- granulating a portion of etoricoxib and excipient or a mixture of excipients using processes known in the art as slugging and/or roller compaction;
- addition of the resting etoricoxib and excipient or a mixture of excipients to the granulate to give compression mixture;
- compressing the compression mixture to the desired form;
- optionally, applying a coating.

In yet another aspect, the wet granulation process comprises:
- granulating an excipient or a mixture of excipients using granulation liquids, based on water, organic solvents or organic/water liquid solvents, which contain less that 50% of water;
- addition of etoricoxib and excipient or a mixture of excipients to the granulate to give compression mixture;
- compressing the compression mixture to the desired form;
- optionally, applying a coating.

In another aspect, the wet granulation process comprises:
- granulating etoricoxib and excipient or a mixture of excipients using granulation liquids, based on water, organic solvents or organic/water liquid solvents, which contain less that 50% of water;
- addition of excipient or a mixture of excipients to the granulate to give compression mixture;
- compressing the compression mixture to the desired form;
- optionally, applying a coating.

In yet another aspect, the wet granulation process comprises:
- granulating a portion of etoricoxib and excipient or a mixture of excipients using granulation liquids, based on water, organic solvents or organic/water liquid solvents, which contain less that 50% of water;
- addition of the resting etoricoxib and excipient or a mixture of excipients to the granulate to give compression mixture;
- compressing the compression mixture to the desired form;
- optionally, applying a coating.

It has been found out that the composition comprising etoricoxib in pure polymorphic form I prepared by the process according to present invention is stable at stability testing conditions, which is confirmed by comparative results to the reference product.

The following examples illustrate the invention. All the formulations were prepared using etoricoxib of polymorphic form I obtained by the process according to present invention.

### Examples

The compounds prepared by the process according to the present invention and other compounds were characterized with regard to their X-ray powder diffraction patterns (obtained by a Phillips PW3040/60 X'Pert PRO diffractometer using CuKα radiation of 1,541874 Ǻ). Images of particles were taken on a Microscope Olympus BX 50 equipped with Olympus camera DP70.

### Comparative Example 1

Etoricoxib (30 g) and isopropyl alcohol (140 ml) are charged into a glass reactor, equipped with condenser and mechanical stirrer. The mixture is heated to 80 °C and maintained until a clear solution is formed. Then the solution is gradually cooled to 58 °C. After the temperature of obtained solution reached the 58 °C, seeds of etoricoxib (Form I, 200 mg) are added and maintained at this temperature until dense suspension is formed (approx. 15 minutes). Obtained suspension is cooled to 20 °C with approx. cooling rate 0.4 °C/min and maintained at this temperature for next 30 minutes. Obtained product is isolated with suction filtration using Büchner funnel. The isolated product is dried in a vacuum dryer under reduced pressure (<100 mbar) and temperature 40 °C for 12 hours.
(yield: 27.0 g, Form I+IV)

### Process for preparing polymorphic form I by primary nucleation

### Example 1

Etoricoxib (12.5 g) and isopropyl alcohol (70 ml) are charged into a glass reactor, equipped with condenser and mechanical stirrer. The mixture is heated to 80 °C and maintained until a clear solution is formed. Then the solution is gradually cooled to 20 °C with average cooling ramp 1.0 °C/min. During the phase of cooling suspension is formed. After the final crystallization temperature (20 °C) is reached the suspension is maintained at this temperature for the next 40 minutes. Obtained product is isolated with suction filtration using Büchner funnel. The isolated product is dried in a vacuum dryer under reduced pressure (<100 mbar) and temperature 40 °C for 10 hours.
(yield: 10.9 g, Form I)

### Example 2

Etoricoxib (12.5 g) and isopropyl alcohol (70 ml) are charged into a glass reactor, equipped with condenser and mechanical stirrer. The mixture is heated to 80 °C and maintained until a clear solution is formed. Then the solution is gradually cooled to 20 °C with average cooling ramp 1.0 °C/min. During the phase of cooling suspension is formed. After the final crystallization temperature (20 °C) is reached the suspension is maintained at this temperature for the next 40 minutes. Obtained product is isolated with suction filtration using Büchner funnel and washed with 7 ml of ice-cold isopropyl alcohol. The isolated product is dried in a vacuum dryer under reduced pressure (<100 mbar) and temperature 40 °C for 10 hours.
(yield: 10.7 g, Form I)

### Process for preparing polymorphic form I by secondary nucleation

### Example 3

Etoricoxib (14 g) and isopropyl alcohol (65 ml) are charged into a glass reactor, equipped with condenser and mechanical stirrer. The mixture is heated to 80 °C and maintained until a clear solution is formed. Then the solution is cooled to 25 °C with average cooling rate 1.5 °C/min. After the temperature of solution reached 54 °C seeds of etoricoxib (Form I, 100 mg) are added and suspension is formed. Obtained suspension is maintained at final crystallization temperature 25 °C for the next 20 minutes. Obtained product is isolated with suction filtration using Büchner funnel. The isolated product is dried in a vacuum dryer under reduced pressure (<100 mbar) and temperature 40 °C for 12 hours.
(yield: 12.5 g, Form I)

### Example 4

Etoricoxib (12.5 g) and isopropyl alcohol (70 ml) are charged into a glass reactor, equipped with condenser and mechanical stirrer. The mixture is heated to 80 °C and maintained until a clear solution is formed. Then the solution is gradually cooled to 52 °C (reactor jacket temperature 50 °C). The obtained solution is further cooled with average cooling rate 1 °C/min to final crystallization temperature, 20 °C. During the cooling, seeds of etoricoxib (Form I, 100 mg) are added at the temperature of solution 47 °C and suspension is formed. After the final crystallization temperature (20 °C) is reached the suspension is maintained at this temperature for the next 40 minutes. Obtained product is isolated with suction filtration using Büchner funnel. The isolated product is dried in a vacuum dryer under reduced pressure (<100 mbar) and temperature 40 °C for 12 hours.
(yield: 11.0 g, Form I)

### Example 5

Etoricoxib (700 g) and isopropyl alcohol (4520 ml) are charged into a glass reactor, equipped with condenser and mechanical stirrer. The mixture is heated to 80 °C and maintained until a clear solution is formed. Then the solution is gradually cooled to 48 °C (reactor jacket temperature 45 °C) and seeds of etoricoxib (Form I, 6.0 g) are added. The obtained suspension is further cooled with average cooling rate 1 °C/min to final crystallization temperature, 15 °C. After the temperature 15 °C is reached the suspension is maintained at this temperature for the next 30 minutes. Obtained product is isolated with suction filtration using Büchner funnel and washed with 200 ml of ice-cold isopropyl alcohol. The isolated product is dried in a vacuum dryer under reduced pressure (<100 mbar) and temperature 40 °C for 4 hours and 45 °C for the next 10 hours.
(yield: 655 g, Form I, LOD = 0.45 %, chromatographic purity 99.77 area% HPLC)

### Example 6

Etoricoxib (750 g) and isopropyl alcohol (4200 ml) are charged into a glass reactor, equipped with condenser and mechanical stirrer. The mixture is heated to 80 °C and maintained until a clear solution is formed. Then the solution is gradually cooled to 47 °C (reactor jacket temperature 45 °C) and seeds of etoricoxib (Form I, 6.0 g) are added. The obtained suspension is further cooled with average cooling rate 1 °C/min to 20 °C. After the final crystallization temperature (20 °C) is reached the suspension is maintained at this temperature for the next 40 minutes. Obtained product is isolated with suction filtration using Büchner funnel and washed with 200 ml of ice-cold isopropyl alcohol. The isolated product is dried in a vacuum dryer under reduced pressure (<100 mbar) and temperature 40 °C for 5 hours and 45 °C for next 10 hours.
(yield: 680 g, Form I, LOD = 0.40 %)

### Example 7

Etoricoxib (3.25 kg) and isopropyl alcohol (24.4 L) are charged into a 60 L reactor (with propeller type stirrer) equipped with reflux-column. The mixture is heated to 70 °C and maintained until a clear solution is formed. Then the solution is cooled to 52 °C with average cooling rate 0.3 °C/min. After the temperature of solution reached 52 °C seeds of etoricoxib (Form I, 15 g) are added and suspension is formed. Obtained suspension is further cooled to final crystallization temperature 15 °C in next 50 minutes, with average cooling rate approx. 0.7 °C/min. After the final crystallization temperature is reached, the suspension is further mixed for the next 45 minutes. Obtained product is isolated with filtration under nitrogen using filter-dryer. The isolated product is dried in a vacuum tray-dryer under reduced pressure (<100 mbar) and temperature 40 °C for 16 hours.
(yield: 2.92 kg, Form I)

### Pharmaceutical Composition Examples

### Examples F1-F8

### Preparation of granulate

The respective solid components shown in the table below were homogenized in a container mixer. The homogenized material was compacted using a roller compactor by applying a compaction force and a roller speed up to 7 rpm. The compacted material was sieved again to prepare sieved granulate.

**Table. Composition of granulate (in mg/1 tablet core).**

| | F1a | F2a | F3a | F4a | F5a | F6a | F7a | F8a |
|---|---|---|---|---|---|---|---|---|
| Component | | | | | | | | |
| Etoricoxib | 60.0 | 60.0 | 60.0 | 60.0 | 60.0 | 60.00 | 60.0 | 60.0 |
| Calcium hydrogen phosphate anhydrous (Anhydrous Emcompress) | 100.0 | - | - | - | 71.0 | 60.00 | - | 60.0 |
| Microcrystalline cellulose (Avicel PH112) | - | 103.0 | - | - | 60.0 | 71.00 | 98.0 | - |
| Mannitol (Partec M200) | - | - | 98.0 | - | - | - | - | - |
| Lactose monohydrate (Tablettose or Pharmatose) | - | - | - | 94.0 | - | - | - | 71.0 |
| Low substituted hydroxypropylcellulose LH-B1 | 15.0 | - | - | - | - | - | - | - |
| Low substituted hydroxypropylcellulose LH-21 | - | 10.0 | - | - | - | - | - | - |
| Low substituted hydroxypropylcellulose LH-11 | - | - | 20.0 | - | - | - | - | - |
| Crospovidone | - | - | - | 30.0 | - | - | - | - |
| Sodium starch glycolate (type A) (Primojel) | - | - | - | - | - | - | 20.0 | - |
| Croscarmellose sodium (Ac-Di-Sol) | - | - | - | - | 3.0 | 3.0 | - | 2.0 |
| Colloidal anhydrous silica | 3.0 | - | - | - | - | - | - | - |
| Magnesium stearate | 2.0 | 2.0 | 2.0 | 1.0 | 1.0 | 1.0 | 2.0 | 1.0 |
| TOTAL | 180.0 | 175.0 | 180.0 | 185.0 | 195.0 | 195.0 | 180.0 | 194.0 |

### Preparation of compression mixtures

The components shown in table below were added to the granulates (F1a)-(F8a) obtained above and mixed in a high-shear mixer (HSM) or conic mixer (CM) to obtain compression mixture.

**Table. Composition of compression mixture (in mg/1 tablet core).**

| | F1b | F2b | F3b | F4b | F5b | F6a | F7b | F8b |
|---|---|---|---|---|---|---|---|---|
| Component | | | | | | | | |
| Granulate | 1a | 2a | 3a | 4a | 5a | 6a | 6a | 7a |
| | 180.0 | 170.0 | 180.0 | 185.0 | 195.0 | 195.0 | 180.0 | 194.0 |
| Microcrystalline cellulose (Avicel PH112) | 15.0 | - | - | 10.0 | - | - | - | - |
| Mannitol (Partec M200) | - | 15.0 | - | - | - | - | - | - |
| Lactose monohydrate (Tablettose or Pharmatose) | - | - | 10.0 | - | - | - | - | - |
| Low substituted hydroxypropylcellulose LH-B1 | - | - | 8.0 | - | - | - | - | - |
| Low substituted hydroxypropylcellulose LH-11 | - | 12.0 | - | - | - | - | - | - |
| Crospovidone (Kollidon CL) | - | - | - | - | - | - | 15.0 | - |
| Croscarmellose sodium (Ac-Di-Sol) | - | - | - | 3.0 | 3.0 | 3.0 | - | 3.0 |
| Colloidal anhydrous silica | 2.0 | - | - | - | - | - | 2.0 | - |
| Magnesium stearate | 3.0 | 3.0 | 2.0 | 2.0 | 2.0 | 2.0 | 3.0 | 3.0 |
| TOTAL | 200.0 | 200.0 | 200.0 | 200.0 | 200.0 | 200.0 | 200.0 | 200.0 |

The physical properties of compression mixture (sieving analysis, bulk and tapped volume, flowability, angle of repose) were determined. The results for the compression mixture Example F5b are as follows:
- sieving analysis (performed on apparatus Hosokawa Alpine 200LS-N):
   ∘ wt. % of particles, passed through the sieve with openings 71 µm: 29.5
   ∘ wt. % of particles, passed through the sieve with openings 125 µm: 49.0
   ∘ wt. % of particles, passed through the sieve with openings 250 µm: 73.0
   ∘ wt. % of particles, passed through the sieve with openings 500 µm: 87.5
   ∘ wt. % of particles, passed through the sieve with openings 710 µm: 98.5
- bulk volume: 1.84 mL/g
- tapped volume (determined on Stampfvolumeter STAV2003): 1.60 mL/g
- flowability (determined by Sotax Flowtest FT300): 43.4 s
- angle of repose (determined by Sotax Flowtest FT300): 39.8°

### Preparation of tablet cores

The compression mixtures (F1b)-(F8b) were compressed into round or oval tablets with theoretical weight of 200.00 mg. The disintegration time of tablet cores was measured on Erweka according to Ph. Eur. In purified water at 37°C. Disintegration time of tablet cores of Example 5 was 15-25 seconds at average hardness (performed by Erweka on 20 tablet cores) 95 N. Average weight of 20 tablet cores was 200.4 mg.

### Film coating

Tablet cores of Example (F1a)-(F8a) were coated in automatic coating pan with water-based film coating suspension, prepared by suspending of ready-to-use mixture, commercially available as Opadry 85F28751 II white. The theoretical weight of the film coated tablets was 206.0 mg.

### Examples F9-F15

### Preparation of granulate

The respective solid components shown in the table below were homogenized and granulated in high-shear mixer/granulator and transferred to fluid-bed dryer or homogenized, granulated and dried in fluid-bed granulator/dryer. The dried material was sieved again to prepare sieved granulate.

**Table. Composition of granulate (in mg/1 tablet core).**

| | F9a | F10a | F11a | F12a | F13a | F14a | F15a |
|---|---|---|---|---|---|---|---|
| Component | | | | | | | |
| Etoricoxib | 60.0 | 60.0 | 60.0 | 60.0 | 60.0 | 60.0 | 60.0 |
| Calcium hydrogen phosphate anhydrous (Anhydrous Emcompress) | 110.0 | | | | 50.0 | | 50.0 |
| Microcrystalline cellulose (Avicel PH112) | 50.0 | | | 75.0 | 110.0 | 75.0 | |
| Mannitol (Partec M200) | | 115.0 | | | | 50.0 | 75.0 |
| Lactose monohydrate (Tablettose or Pharmatose) | | | 105.0 | 75.0 | | | |
| Povidone | 4.0 | | 5.0 | | | 5.0 | |
| Hydroxypropylcellulos e (Klucel EF) | | 5.0 | | 5.0 | 5.0 | | 5.0 |
| Low substituted hydroxypropylcellulose | | 20.0 | | | | 10.0 | |
| Sodium starch glycolate (type A) (Primojel) | | | 10.0 | | | | |
| Croscarmellose sodium (Ac-Di-Sol) | | | | 5.0 | 5.0 | | |
| Methanol | | q.s. | | | | | |
| Ethanol | | | q.s. | | | | |
| Ethanol/purified water (90:10 wt./wt%) | | | | q.s. | | | |
| Isopropanol | q.s. | | | | q.s. | | |
| TOTAL | 224.0 | 200.0 | 180.0 | 220.0 | 230.0 | 200.0 | 190.0 |

### Preparation of compression mixtures

The components shown in table below were added to the granulates (F9a)-(F15a) obtained above and mixed in a high-shear mixer (HSM) or conic mixer (CM) to obtain compression mixture.

**Table. Composition of compression mixture (in mg/1 tablet core).**

| | F9b | F10b | F11b | F12b | F13b | F14b | F15b |
|---|---|---|---|---|---|---|---|
| Component | | | | | | | |
| Granulate | 8a | 9a | 10a | 11a | 12a | 13a | 14a |
| | 224.0 | 200.0 | 180.0 | 220.0 | 230.0 | 200.0 | 190.0 |
| Calcium hydrogen phosphate anhydrous (Anhydrous Emcompress) | - | - | - | 10.0 | - | - | - |
| Microcrystalline cellulose (Avicel PH112) | - | 24.0 | 36.0 | - | - | - | - |
| Mannitol (Partec M200) | - | - | - | - | - | 27.0 | - |
| Lactose monohydrate (Tablettose or Pharmatose) | - | - | - | - | - | - | 33.0 |
| Low substituted hydroxypropylcellulose LH-11 | - | - | 15.0 | - | - | - | 10.0 |
| Sodium Starch Glycolate (type A) (Primojel) | - | 10.0 | - | 5.0 | - | 5.0 | - |
| Croscarmellose sodium (Ac-Di-Sol) | 10.0 | - | - | - | 5.0 | - | - |
| Colloidal anhydrous silica | - | - | 2.0 | - | - | 2.0 | - |
| Magnesium stearate | - | 4.0 | 5.0 | 3.0 | - | - | 5.0 |
| Calcium stearate | 4.0 | - | - | - | 3.0 | 4.0 | - |
| TOTAL | 238.0 | 238.0 | 238.0 | 238.0 | 238.0 | 238.0 | 238.0 |

The physical properties of compression mixture (sieving analysis, bulk and tapped volume, flowability, angle of repose) were determined. The results for the compression mixture Example 9b are as follows:
- sieving analysis (performed on apparatus Hosokawa Alpine 200LS-N):
   ∘ wt. % of particles, passed through the sieve with openings 71 µm: 22.0
   ∘ wt. % of particles, passed through the sieve with openings 125 µm: 37.5
   ∘ wt. % of particles, passed through the sieve with openings 250 µm: 79.0
   ∘ wt. % of particles, passed through the sieve with openings 500 µm: 91.5
   ∘ wt. % of particles, passed through the sieve with openings 710 µm: 97.5
- bulk volume: 1.40 mL/g
- tapped volume (determined on Stampfvolumeter STAV2003): 1.16 mL/g
- flowability (determined by Sotax Flowtest FT300): 28.7 s
- angle of repose (determined by Sotax Flowtest FT300): 32.4°

### Preparation of tablet cores

The compression mixtures (F9b)-(F15b) were compressed into round or oval tablets with theoretical weight of 238.00 mg. The disintegration time of tablet cores was measured on Erweka according to Ph. Eur. In purified water at 37°C. Disintegration time of tablet cores of Example F9 was 75 seconds at average hardness (performed by Erweka on 20 tablet cores) 86 N. Weight of 20 tablet cores was from 238.8 to 242.8 mg.

### Film coating

Tablet cores of Example (F8a)-(F14a) were coated in automatic coating pan with water-based film coating suspension, prepared by suspending of ready-to-use mixture, commercially available as Opadry 85F28751 II white. The theoretical weight of the film coated tablets was 244.0 mg.

### Examples F16-F22

### Preparation of direct compression mixture

The components shown in table below were mixed in a high-shear mixer (HSM) or conic mixer (CM) to obtain compression mixture.

**Table. Composition of direct compression mixture (in mg/1 tablet core).**

| | F16 | F17 | F18 | F19 | F20 | F21 | F22 |
|---|---|---|---|---|---|---|---|
| Component | | | | | | | |
| Etoricoxib | 60.0 | 60.0 | 60.0 | 60.0 | 60.0 | 60.0 | 60.0 |
| Calcium hydrogen phosphate anhydrous (Anhydrous Emcompress) | - | 40.0 | - | - | - | - | - |
| Microcrystalline cellulose (Avicel PH112) | - | 77.0 | - | 60.0 | 127.0 | - | 70.0 |
| Mannitol (Partec M200) | 112.0 | - | - | - | - | 60.0 | 47.0 |
| Sorbitol | - | - | - | - | - | 55.0 | - |
| Lactose monohydrate (Tablettose or Pharmatose) | - | - | 118.0 | 57.0 | - | - | - |
| Povidone | - | - | 6.0 | - | - | - | - |
| Low substituted hydroxypropylcellulose LH-11 | 20.0 | - | - | 15.0 | - | 15.0 | - |
| Sodium starch glycolate (type A) (Primojel) | - | 10.0 | - | - | - | - | 15.0 |
| Croscarmellose sodium (Ac-Di-Sol) | - | - | 6.0 | - | 5.0 | - | - |
| Colloidal anhydrous silica | - | 2.0 | 2.0 | - | - | 2.0 | - |
| Magnesium stearate | 3.0 | 6.0 | 3.0 | 3.0 | 3.0 | 3.0 | 3.0 |
| TOTAL | 195.0 | 195.0 | 195.0 | 195.0 | 195.0 | 195.0 | 195.0 |

The physical properties of compression mixture (sieving analysis, bulk and tapped volume, flowability, angle of repose) were determined. The results for the compression mixture Example F17 are as follows:
- sieving analysis (performed on apparatus Hosokawa Alpine 200LS-N):
   ∘ wt. % of particles, passed through the sieve with openings 71 µm: 19.5
   ∘ wt. % of particles, passed through the sieve with openings 125 µm: 60.5
   ∘ wt. % of particles, passed through the sieve with openings 250 µm: 98.0
   ∘ wt. % of particles, passed through the sieve with openings 500 µm: 100.0
- bulk volume: 2.70 mL/g
- tapped volume (determined on Stampfvolumeter STAV2003): 1.84 mL/g
- flowability (determined by Sotax Flowtest FT300): 161.4 s
angle of repose (determined by Sotax Flowtest FT300): 40.8°

### Preparation of tablet cores

The compression mixtures (F16)-(F22) were compressed into round or oval tablets with theoretical weight of 195.00 mg. The disintegration time of tablet cores was measured on Erweka according to Ph. Eur. In purified water at 37°C. Disintegration time of tablet cores of Example F17 was 15 seconds at average hardness (performed by Erweka on 20 tablet cores) 97 N. Weight of 20 tablet cores was from 185.4 to 207.0 mg.

### Film coating

Tablet cores of Example (F16)-(F22) were coated in automatic coating pan with water-based film coating suspension, prepared by suspending of ready-to-use mixture, commercially available as Opadry 85F28751 II white. The theoretical weight of the film coated tablets was 200.0 mg.

### Examples F23-26

### Preparation of granulate

The respective solid components shown in the table below were homogenized in a container mixer. The homogenized material was compacted using a roller compactor Fitzpatrick IR220A by applying a compaction force and a roller speed up to 7 rpm. The compacted material was sieved again to prepare sieved granulate.

**Table. Composition of granulate (in mg/1 tablet core).**

| | F23a | F24a | F25a | F26a |
|---|---|---|---|---|
| Component | | | | |
| Etoricoxib | 120.0 | 120.0 | 120.0 | 120.0 |
| Calcium hydrogen phosphate anhydrous (Anhydrous Emcompress) | 120.0 | 120.0 | 120.0 | 120.0 |
| Microcrystalline cellulose (Avicel PH112) | 146.0 | 142.0 | 146.0 | 146.0 |
| Croscarmellose sodium (Ac-Di-Sol) | 8.0 | 6.0 | 4.0 | / |
| Magnesium stearate | 2.0 | 2.0 | 2.0 | 2.0 |
| TOTAL | 396.0 | 390.0 | 392.0 | 388.0 |

| | | | | |
|---|---|---|---|---|
| Formulation F24 is linear increase of the composition F6. | | | | |

The particle size distribution of etoricoxib of F24 was prepared using the following particle size distribution determined as described in the present application:

| | F24a/A | F24a/B |
|---|---|---|
| Average particle size | 17 µm | 80 µm |
| D10 | 2.6 µm | 7.8 µm |
| D50 | 11.6 µm | 31.2 µm |
| D90 | 38.5 µm | 190.5 µm |

No impact of particle size of etoricoxib on the technological process was observed during process of roller compaction.

### Preparation of compression mixtures

The components shown in table below were added to the granulates (F23a)-(F26a) obtained above and mixed in a high-shear mixer (HSM) or conic mixer (CM) to obtain compression mixture.

**Table. Composition of compression mixture (in mg/1 tablet core).**

| | F23b | F24b | F25b | F26b |
|---|---|---|---|---|
| Component | | | | |
| Granulate | 23a 396.0 | F24a 390.0 | F25a 392.0 | F26a 388.0 |
| Croscarmellose sodium (Ac-Di-Sol) | | 6.0 | 4.0 | 8.0 |
| Magnesium stearate | 4.0 | 4.0 | 4.0 | 4.0 |
| TOTAL | 400.0 | 400.0 | 400.0 | 400.0 |

The physical properties of compression mixture (sieving analysis, bulk and tapped volume, flowability, angle of repose) were determined (performed on apparatus Hosokawa Alpine 200LS-N): The results for the compression mixture, using average particle sizes of etoricoxib 17 µm and 80 µm for the Examples F24bA and F24bB, respectively:

| | F24bA | F24bB |
|---|---|---|
| Sieving analysis (wt. % of particles, passed through the sieve with openings: | | |
| 71 µm | 20.5 | 19.0 |
| 125 µm | 38.0 | 33.5 |
| 250 µm | 67.0 | 62.0 |
| 500 µm | 86.0 | 84.0 |
| 710 µm | 93.5 | 97.0 |
| Bulk volume (mL/g) | 1.84 | 1.62 |
| Tapped volume (determined on Stampfvolumeter STAV2003) (mL/g) | 1.36 | 1.26 |
| Flowability (determined by Sotax Flowtest FT300) (s) | 34.7 | 31.1 |
| Angle of repose (determined by Sotax Flowtest FT300) (°) | 43.1 | 43.2 |

### Preparation of tablet cores

The compression mixtures (F23b)-(F26b) were compressed into round or oval tablets with theoretical weight of 400.00 mg. The disintegration time of tablet cores was measured on Erweka according to Ph. Eur. In purified water at 37°C.

Disintegration time of tablet cores was 18 seconds at average hardness (calculated on 20 tablet cores) 108 N, and 25 seconds at average hardness (calculated on 20 tablet cores) 102 N for Examples 24A (prepared from compression mixture F24bA) and F24B (prepared from compression mixture F24bB), respectively.

### Film coating

Half of tablet cores of Examples (F23)-(F26) were coated in automatic coating pan with water-based film coating suspension, prepared by suspending of ready-to-use mixture, commercially available as Opadry II white with possible addition of colouring agent(s) (e.g. ferric oxides). The theoretical weight of the film coated tablets was 206.0 mg.

The rest of tablet cores of Examples (F23)-(F26) were coated in automatic coating pan with water-based film coating suspension, prepared by suspending hypromellose, titanium dioxide (E171) and macrogol 4000 with possible addition of colouring agent(s) (e.g. ferric oxides). The theoretical weight of the film coated tablets was 206.0 mg.

### Results of stress stability testing

Film coated tablets of Examples F5 (dry granulation), F9 (wet granulation) and F17 (direct compression) were compared to the reference product Arcoxia 60 mg. All the samples have been subjected to different stress stability conditions. The results of the increased total impurities in comparison to the initial results are presented in the table below.

**Table. Results of the stress stability testing (increase of total impurities in comparison to the initial results).**

| Stress stability condition | Example F5 | Example F9 | Example F17 | Arcoxia 60 mg |
|---|---|---|---|---|
| 40°C/75% relative humidity opened after 1 month | 0.00% | 0.00% | 0.00% | 0.00% |
| 40°C/75% relative humidity opened after 4 month | 0.00% | - | - | 0.00% |
| 40°C/75% relative humidity closed after 1 month | 0.00% | 0.00% | 0.00% | 0.00% |
| 40°C/75% relative humidity closed after 4 month | 0.00% | - | - | 0.01% |
| 50°C/75% relative humidity closed after 1 month | 0.00% | 0.00% | 0.00% | 0.00% |

It is obvious to those skilled in art that stress stability testing of the formulations comprising etoricoxib of polymorphic form I, prepared by different technological processes, e.g. dry granulation, wet organic granulation and direct compression, confirm the chemical stability of the formulations in comparison to the reference product, which is surprisingly in the view of the prior art documents teaching that stability of the etoricoxib in polymorphic form I is very omitted and difficult to assure.

Furthermore, also physical stability of the formulation of Example F5 was compared to Arcoxia 60 mg. Besides formulation of Example F5, also formulation of Example F5' was prepared. The qualitative and quantitative composition of both formulations is the same; the only difference is in the physical form of etoricoxib incorporated into the formulation: whereas Example F5 comprises etoricoxib in pure polymorphic form I, and formulation of Example F5' comprises etoricoxib in polymorphic form I with traces of hemihydrate.

The results of stability testing are summarized in the table below:

| Stress stability conditions | Etoricoxib Form I | Etoricoxib in Form I with traces of hemihydrate | Example F5 Form I | Example F5' Form I with trace of hemihydrate | Arcoxia 60 mg Form V |
|---|---|---|---|---|---|
| 40°C/75% relative humidity closed after 1 month | No change | No change | No change | No change | No change |
| 40°C/75% relative humidity open after 1 month | No change | Content of hemihydrate increased | No change | Content of hemihydrate increased | Content of hemihydrate increased |

In Figure 1 a comparison of diffraction patterns of etoricoxib polymorphic form I (curve 1) with diffraction patterns of the samples of etoricoxib polymorphic form I, stored at 40°C/75%RH for 1 month in open and closed container is shown (curve 2,3). As evident from the figure there is no change in characteristic diffraction pattern of polymorphic form I even after storage at 40°C / 75 %RH for 1 month in open or closed container, which proves that the polymorphic form prepared by the process according to present invention is surprisingly stable.

Figure 2 shows the diffraction pattern of etoricoxib which contains (1) mixture of polymorphic form I and trace of hemihydrate form of etoricoxib. Diffraction patterns of the samples, stored at 40°C/75%RH in open and closed container (2,3) in comparison with characteristic diffraction pattern of mixture of polymorphic form of API (1) prove that the content of the hemihydrate form is increased after storage at 40°C / 75 %RH for 1 month in open container (Fig. 2, curve (3)). The content of hemihydrate form of etoricoxib is not increased after storage at 40 °C / 75 %RH for 1 month in closed container (Fig. 2, curve (2)).

Evidently, etoricoxib containing even small amounts of hemihydrate form would convert during time to undesired physical form, whereas pure polymorphic form I has no tendency to conversion. Consequently, pure polymorphic form I is preferably used for incorporation into pharmaceutical compositions.

In Figure 3 X-ray powder diffraction patterns of etoricoxib tablets, comprising 120 mg of polymorphic form I, of the initial state (1), state after storage at 40°C/75%RH for 1 month in closed and open container (2, 3) are compared.
As evident from the Figure 3 the powder diffraction pattern of initial tablet (curve 1) and tablet after stability testing (curve 2, 3) at 40°C/75%RH for 1 month did not change, thus it could be concluded that polymorphic form I prepared by the process according to present invention is surprisingly stable also when incorporated into pharmaceutical composition and subjected to extreme conditions.
In contrast to this a presence of even trace amounts of hemihydrate form in the active ingredient when incorporated into the pharmaceutical dosage form would catalyse conversion of polymorphic form I to hemihydrate form which is evident from the figure 4.
In Figure 4 X-ray powder diffraction patterns of etoricoxib tablets comprising 120 mg of etoricoxib in polymorphic form I with trace of hemihydrate form (initial sate (1), state after storage at 40°C/75%RH for 1 month in closed and open container (2, 3)) are depicted.

X-ray powder diffraction patterns of the tablet in initial state (curve 1) and the tablet after stability testing (curve 2) at 40°C/75%RH for 1 month in closed container are identical, no increase in the content of hemihydrate form is observed. X-ray powder diffraction pattern of drug product stored under the same condition in open container (3) shows an increase in the content of etoricoxib hemihydrate form.

In Figure 5 X-ray powder diffraction patterns of etoricoxib tablets 60 mg -Arcoxia: initial sate(1), state after storage at 40°C/75%RH for 1 month in closed (2) and open container (3), diffraction pattern of API (etoricoxib form V (4) and diffraction pattern of etoricoxib hemihydrate form (5) are presented.

The drug product in initial state contains polymorphic form V of etoricoxib (1). The X-ray powder diffraction patterns of drug product (Figure 4) and drug product after stability testing at 40°C/75%RH for 1 month in closed container (2) are identical. (There is no increase in the content of etoricoxib hemihydrate form). However, X-ray powder diffraction pattern of drug product stored under the same condition in open container (3) presents an increase in the content of etoricoxib hemihydrate form.

## Claims

1. A process for the preparation of crystallographically essentially pure polymorphic form I of etoricoxib which comprises:
(i) dissolving etoricoxib in a suitable solvent system at a temperature in the range of from 50°C to refluxing temperature of said organic solvent system;
(ii) optionally filtering the obtained crystallization mixture;
(iii) cooling the crystallization mixture to a nucleation temperature in the range of from 30°C to 56°C,
(iv) then cooling the crystallization mixture to a temperature in the range of from - 20°C to 28°C,
(v) separating and drying the crystallized polymorphic form I of etoricoxib;
wherein the term crystallographically essentially pure means that the polymorphic form comprises less than 2 wt % of other polymorphic forms, amorphous form or solvates, wherein polymorphic purity is determined by means of X-ray powder diffraction, wherein polymorphic form I of etoricoxib is **characterized by** an X-ray diffraction pattern obtained using a Cu K alpha source and comprising signals with peak positions in accordance with Figure 1; and wherein the solvent system is 2-propanol.

2. A process according to claim 1 wherein in the step (iii) solution of etoricoxib in a suitable solvent system is gradually cooled down to a nucleation temperature of the crystallization mixture maintaining a controlled cooling profile with an average cooling rate within 0.1 to 2.5°C/min.

3. A process according to any preceding claim wherein nucleation temperature of the crystallization mixture is selected within the range of 45° to 55°C.

4. A process according to any preceding claim wherein nucleation is induced by primary and/or secondary nucleation mechanism.

5. A process according to any preceding claim wherein nucleation is induced by adding seeds of pure polymorphic form I of etoricoxib.

## Patentansprüche

1. Verfahren zur Herstellung der kristallographisch im Wesentlichen reinen polymorphen Form I von Etoricoxib, umfassend:
(i) Lösen von Etoricoxib in einem geeigneten Lösungsmittelsystem bei einer Temperatur im Bereich von 50°C bis zur Rückflusstemperatur des organischen Lösungsmittelsystems;
(ii) gegebenenfalls Filtrieren der erhaltenen Kristallisationsmischung;
(iii) Abkühlen der Kristallisationsmischung auf eine Keimbildungstemperatur im Bereich von 30°C bis 56°C,
(iv) anschließendes Abkühlen des Kristallisationsgemisches auf eine Temperatur im Bereich von -20°C bis 28°C,
(v) Abtrennen und Trocknen der kristallisierten polymorphen Form I von Etoricoxib;
worin der Begriff kristallographisch im Wesentlichen rein bedeutet, dass die polymorphe Form weniger als 2 Gew.-% anderer polymorpher Formen, amorpher Formen oder Solvate umfasst, worin die polymorphe Reinheit mittels Röntgenpulverbeugung bestimmt wird, worin die polymorphe Form I von Etoricoxib durch ein Röntgenbeugungsmuster gekennzeichnet ist, das unter Verwendung einer Cu-K-alpha-Quelle erhalten wurde und Signale mit Peakpositionen gemäß Fig. 1 umfasst, und worin das Lösungsmittelsystem 2-Propanol ist.

2. Verfahren gemäß Anspruch 1, worin in Schritt (iii) die Lösung von Etoricoxib in einem geeigneten Lösungsmittelsystem schrittweise auf eine Keimbildungstemperatur des Kristallisationsgemisches heruntergekühlt wird, wobei ein kontrolliertes Kühlprofil mit einer durchschnittlichen Abkühlgeschwindigkeit innerhalb von 0,1 bis 2,5°C/min aufrechterhalten wird.

3. Verfahren gemäß mindestens einem der vorhergehenden Ansprüche, worin die Keimbildungstemperatur des Kristallisationsgemisches im Bereich von 45°C bis 55°C ausgewählt ist.

4. Verfahren gemäß mindestens einem der vorhergehenden Ansprüche, worin die Keimbildung durch einen primären und/oder sekundären Keimbildungsmechanismus induziert wird.

5. Verfahren gemäß mindestens einem der vorhergehenden Ansprüche, worin die Keimbildung durch Zugabe von Keimen der reinen polymorphen Form I von Etoricoxib induziert wird.

## Revendications

1. Procédé pour la préparation d'une forme polymorphique I cristallographiquement essentiellement pure d'étoricoxib qui comprend :
(i) la dissolution d'étoricoxib dans un système de solvant approprié à une température dans le domaine allant de 50°C à la température de reflux dudit système de solvant organique ;
(ii) optionnellement la filtration du mélange de cristallisation obtenu ;
(iii) le refroidissement du mélange de cristallisation à une température de nucléation dans le domaine allant de 30°C à 56°C,
(iv) ensuite le refroidissement du mélange de cristallisation à une température dans le domaine allant de -20°C à 28°C,
(v) la séparation et le séchage de la forme polymorphique I d'étoricoxib cristallisée ;
dans lequel le terme cristallographiquement essentiellement pure signifie que la forme polymorphique comprend mois de 2% en poids d'autres formes polymorphiques, forme amorphes ou solvates, dans lequel la pureté polymorphique est déterminée par diffraction de rayons X sur poudre, dans lequel la forme polymorphique I d'étoricoxib est **caractérisée par** un spectre de diffraction de rayons X obtenu en utilisant une source alpha de Cu K et comprenant des signaux avec des positions de pic en accordance avec la Figure 1; dans lequel le système de solvant est le 2-propanol.

2. Procédé selon la revendication 1 dans lequel dans l'étape (iii) la solution d'étoricoxib dans un système de solvant approprié est graduellement refroidi jusqu'à une température de nucléation du mélange de cristallisation en maintenant un profil de refroidissement contrôlé avec une vitesse de refroidissement moyenne dans le domaine de 0,1 à 2,5°C/min.

3. Procédé selon l'une quelconque des revendications précédentes dans lequel la température de nucléation du mélange de cristallisation est choisie dans le domaine de 45°C à 55°C.

4. Procédé selon l'une quelconque des revendications précédentes dans lequel la nucléation est induite par un mécanisme de nucléation primaire et/ou secondaire.

5. Procédé selon l'une quelconque des revendications précédentes dans lequel la nucléation est induite en ajoutant des semences de forme polymorphique I d'étoricoxib pure.
